# EUROPEAN PATENT APPLICATION

(11) **EP 4 119 658 A1**
(43) Date of publication of application: **18.01.2023**
(21) Application number: 21186223.0
(22) Date of filing: 16.07.2021
(51) Int. Cl.: C12N 9/16, A23K 10/16

(54) **PHYTASE VARIANTS**

(71) Applicant: AB Enzymes Oy, 05200 Rajamäki (FI)
(72) Inventor: JUNTUNEN, Kari, 05200 Rajamäki (FI); AHOLA, Pihla, 05200 Rajamäki (FI); BENSON, Sven, 70569 Stuttgart (DE); LORENZ, Lenz, 70569 Stuttgart (DE); METZGER, Tara, 64293 Darmstadt (DE); KÜHN, Imke, 64293 Darmstadt (DE); PURANEN, Terhi, 05200 Rajamäki (FI); PALOHEIMO, Marja, 05200 Rajamäki (FI)
(74) Representative: Espatent Oy

(57) **Abstract**

A phytase variant with improved IP4 degradation activity, a method for its manufacturing, an animal feed, a feed supplement, a dry and a liquid formulation, a method of degrading phytic acid, a method of manufacturing a phytase variant, a method of manufacturing a feed pellet, and a recombinant host cell configured to produce at least one polypeptide are disclosed, wherein the phytase variant comprises amino acid substitutions and has at least 80% amino acid sequence identity with amino acids of SEQ ID NO: 1.

## Description

### TECHNICAL FIELD

The present disclosure generally relates to field of enzyme technology. The disclosure relates particularly, though not exclusively, to phytase variants having an improved IP4 degradation activity. The present phytase variants are useful in various applications where degradation of phytate is desired, such as in feedstuffs.

### BACKGROUND

This section illustrates useful background information without admission of any technique described herein representative of the state of the art.

Phytic acid (phytate, inositol hexakisphosphate, IP6) is found in many plants where it functions as storage of phosphate. Phosphate stored in IP6 molecules can be released as inorganic phosphate. When inorganic acid is released from myo-inositol hexakisphosphate (IP6) it is converted first to myo-inositol pentakisphosphate (IP5) and further via myo-inositol tetrakisphosphate (IP4), myo-inositol trisphosphate (IP3), myo-inositol bisphosphate (IP2) to myo-inositol monophosphate (IP1).

Phytases are a group of phosphatase enzymes that catalyse the hydrolysis of phytic acid. Commercially available phytases belong to the histidine acid phosphatase (HAP) protein family. The phytases belonging to the HAP protein family share conserved N-terminal active site hepta-peptide motif RHGXRXP and the catalytically active HD-dipeptide at the C-terminus. Histidine acid phosphatases are part of a larger superfamily of histidine phosphatases. They share a conserved catalytic core centred on a histidine, which becomes phosphorylated during the reaction. PFAM motif His_Phos_2 (PF00328) represents branch 2 of the histidine phosphatase superfamily, the branch containing mainly acid phosphatases and phytases.

Phytases are used in feeds to improve phosphate availability from feed ingredients of plant origin (e.g., wheat, barley, corn, soybean) by phytate degradation. This is in particular of interest for monogastric animals like poultry, fish and pigs, because intestinal phytate degradation in the upper intestinal tract of these animals is limited. This limitation not only restricts utilisation of phosphorus, but also the availability of other nutrients due to the chelating effect of inositol phosphates (IPs), particularly by the high IPs, that is IP6, IP5 and IP4. Consequently, at least IP6 to IP4 should be dephosphorylated as completely as possible, but the dephosphorylation should preferably continue even to IP1 to further increase availability of phosphate and other nutrients.

Phytate breakdown by phytases is associated with stepwise degradation of IP6 to lower inositol phosphate esters (IP5, IP4, IP3, IP2, and IP1). The use of industry standard levels of phytase have, expectedly, shown to significantly reduce IP6 levels in vitro and in vivo. However, with IP6 degradation an increase of IP4 and IP3 has been detected in ileal digesta which shows that the hydrolytic cleavage of the first phosphate group is not the only limiting step in phytate degradation (e.g., Zeller et al, 2015; Menezes- Blackburn et al, 2015). As even these lower inositol phosphates have antinutritive properties due to binding of different nutrients like minerals (Xu et al, 1992), the target in animal feeding is to degrade IP esters up to the terminal ileum (Bedford and Walk, 2016). Another reason for the aim to get inositol fully released is that increasing the content of free inositol has been shown to improve growth performance in animals by different mechanisms, still under investigation (Lee and Bedford, 2016).

The part in the intestinal tract of animals where optimal degradation of inositol phosphate esters takes place with phytase supplements is the stomach due to its low pH, leading to the best substrate (phytate) solubility. Retention time in the stomach is short and part of the content might flush rapidly to the intestinal tract in which the pH is neutral. Therefore, further development of phytases acting quickly and more effectively on IP6 and, also on lower inositol phosphates like IP4 and IP3 isomers, is of relevance for the animal feeding industry. These type of phytases would further improve intestinal availability of phosphate and inositol.

It is an object of the present disclosure to provide phytase variants that exhibit phytase activity and that have improved properties that allow their use in industrial processes. Yet another object of the present disclosure is to provide phytase variants that can be used in enzyme compositions for phytate degradation.

### SUMMARY

The appended independent claims define the scope of protection. Any example or technical description of a product and/or method in the description and/or drawings not covered by the claims is presented herein not as an embodiment of the invention, but as background art or as an example which is useful for understanding the claimed invention.

According to a first aspect is provided a variant polypeptide comprising an amino acid sequence having at least 80% amino acid sequence identity with SEQ ID NO: 1, wherein the variant has:
an amino acid substitution at the position 126;
phytase activity; and
wherein the amino acid numbering corresponds to the amino acid numbering of the SEQ ID NO: 1.

Advantageously, the present variant polypeptide (also called herein a phytase variant) has improved IP4 activity compared to the parent phytase having the SEQ ID NO: 1, and/or a phytase having the SEQ ID NO: 2. The position 126 has been identified by the inventors to be important for the increased IP4 activity. Improved IP4 degrading activity of the present phytase variant means that the variant is capable of degrading more IP4 than a phytase with the amino acid sequence of SEQ ID NO: 2. The improved IP degrading activity can be used to an advantage when degradation of phytic acid to lower inositol phosphates is needed, such as in feed or food applications. As shown in the examples provided below, the claimed phytase variant has a IP4 degrading activity that is improved compared to the parent phytase enzyme and/or to a phytase having the SEQ ID NO: 2.

In an embodiment the present variant polypeptide has less than 100% sequence identity with the SEQ ID NO: 1.

In the present variant polypeptide the position 126 is substituted to another amino acid, such as to a histidine, and additionally at least one further amino acid is substituted. This results into an increase in the IP4 degrading activity. The at least one further amino acid substitution, which is substitute in addition to the substitution in position 126, can be selected from a position shown in Table 1. More preferably, the at least one further amino acid substitution is selected from Table 1 such that at least one property selected from IP4 degrading activity, IP6 degrading activity, both IP4 and IP6 degrading activities, and IP4/IP6, is improved for a variant having said substitution, even when present in the variant polypeptide together with other substitutions, as compared to the parent phytase enzyme and/or to a phytase having the SEQ ID NO: 2. The IP6 and IP4 degrading activity can be measured and compared as described in the examples.

In an embodiment the present phytase variant has at least IP4 degrading activity.

In an embodiment the present phytase variant has at least IP6 degrading activity.

In another embodiment the variant polypeptide has IP4 degrading activity and IP6 degrading activity.

In another embodiment the ratio of the variant's IP4 degrading activity to its IP6 degrading activity is at least about 1.05, or within the range 1-2, such as within the range 1.05-2, preferably within the range 1.05-1.9, more preferably within the range 1.2-2 or within the range 1.2-1.9 compared to the IP4/IP6 ratio of a phytase having the SEQ ID NO: 2. In another embodiment the IP4/IP6 ratio is about 1.5, or about 1.6, or about 1.7, or about 1.8 compared to the IP4/IP6 ratio of a phytase having the SEQ ID NO: 2. The larger IP4/IP6 indicates capability to degrade IPs into lower IPs, which is useful in feed and food applications.

In an embodiment is provided a functional fragment of the present phytase variant, the functional fragment having phytase activity.

In an embodiment the variant polypeptide has a histidine at the position 126, i.e., the position 126 may be substituted to a histidine.

In an embodiment the variant polypeptide comprises at least one further amino acid substitution at a position selected from 121, 204, 212, 216, and 258. The substitution may be made to the variant polypeptide artificially, such as by genetic engineering.

In an embodiment the variant polypeptide is an *E*. *coli* 6-phytase. According to this embodiment the amino acid sequence of the present variant polypeptide is derived from an *E*. *coli* 6-phytase, or from its variant.The amino acid in position 126 may naturally be histidine, or it is substituted to histidine from another amino acid present in the *E*. *coli* 6-phytase.

In an embodiment the at least one further amino acid substitution results into the presence of at least one of the following amino acids: 121K, 204N/T, 212G, 216T, and 258N/A. The variant polypeptide is thus modified such that it contains at least one amino acid as specified above.

In an embodiment the at least one further amino acid substitution is a substitution selected from P121K, D204N/T, S212G, M216T, and Q258N/A.

In an embodiment the variant polypeptide comprises the amino acids:
a. 126H; and
b. at least one amino acid selected from: 121K, 204N/T, 212G, 216T, and 258N/A. In an embodiment the variant polypeptide comprises the amino acid substitutions:
   a. N126H; and
   b. at least one amino acid substitution from: P121K, D204N/T, S212G, M216T, and Q258N/A.

In an embodiment the variant polypeptide comprises the amino acid substitutions P121K, N126H, D204N, W211V, S212G, M216T, and V253Y.

In an embodiment the variant polypeptide comprises the amino acid substitutions P121K, N126H, D204T, W211V, S212G, M216T, and V253Y.

In an embodiment the variant polypeptide comprises the amino acid substitutions P121K, N126H, D204N/T, W211V, S212G, M216T, V253Y, and Q258N/A.

In an embodiment the variant polypeptide polypeptide comprises:
a. the amino acids 126H and 204N/T, preferably the substitutions N126H and D204N/T; or
b. the amino acids 126H and 212G, preferably the substitutions N126H and S212G; or
c. the amino acids 126H and 258A/N, preferably the substitutions N126H and Q258A/N; or
d. the amino acids 126H, 204N, and 212G, preferably the substitutions N126H, D204N, and S212G; or
e. the amino acids 126H, 204N, and 258N, preferably the substitutions N126H, D204N, and Q258N; or
f. the amino acids 126H, 204T, and 212G, preferably the substitutions N126H, D204T, and S212G; or
g. the amino acids 126H, 212G, and 258N, preferably the substitutions N126H, S212G, and Q258N; or
h. the amino acids 121K, 126H, and 216T, preferably the substitutions P121K, N126H, and M216T; or
i. the amino acids 126H, 204T, 212G, and 258N, preferably the substitutions N126H, D204T, S212G, and Q258N; or
j. the amino acids 121K, 126H, 204N, 212G, and 216T, preferably the substitutions P121K, N126H, S212G, and M216T.

In an embodiment the variant polypeptide comprises the substitutions selected from:
a. the amino acids 126H, 204N, 258N, preferably the substitutions N126H, D204N, and Q258N; or
b. the amino acids 126H, 204T, and 212G, preferably the substitutions N126H, D204T, and S212G; or
c. the amino acids 126H, 212G, and 258A, preferably the substitutions N126H, S212G, and Q258A; or
d. the amino acids 121K, 126H, 204T, and 216T, preferably the substitutions P121K, N126H, D204T, and M216T; or
e. the amino acids 121K, 126H, 216T, and 258N, preferably the substitutions P121K, N126H, M216T, and Q258N; or
f. the amino acids 126H, 204N, 212G, and 258A, preferably the substitutions N126H, D204N, S212G, and Q258A; or
g. the amino acids 126H, 204T, 212G, 258N, preferably the substitutions N126H, D204T, S212G, and Q258N; or
h. the amino acids 121K, 126H, 204N, 212G, and 216T, preferably the substitutions P121K, N126H, S212G, and M216T; or
i. the amino acids 121K, 126H, 204N, 216T, and 258A, preferably the substitutions P121K, N126H, D204N, M216T, and Q258A; or
j. the amino acids 121K, 126H, 204N, 216T, and 258N, preferably the substitutions P121K, N126H, D204N, M216T, and Q258N; or
k. the amino acids 121K, 126H, 204T, 212G, and 216T, preferably the substitutions P121K, N126H, D204N, S212G, and M216T; or
l. the amino acids 121K, 126H, 204T, 216T, and 258A, preferably the substitutions P121K, N126H, D204T, M216T, and Q258A; or
m. the amino acids 121K, 126H, 204T, 216T, and 258N, preferably the substitutions P121K, N126H, D204T, M216T, and Q258N; or
n. the amino acids 121K, 126H, 212G, 216T, and 258A, preferably the substitutions P121K, N126H, S212G, M216T, and Q258A; or
o. the amino acids 121K, 126H, 212G, 216T, and 258N, preferably the substitutions P121K, N126H, S212G, M216T, and Q258N; or
p. the amino acids 121K, 126H, 204N, 212G, 216T, and 258A, preferably the substitutions P121K, N126H, D204N, S212G, M216T, and Q258A; or
q. the amino acids 121K, 126H, 204T, 212G, 216T, and 258A, preferably the substitutions P121K, N126H, D204T, S212G, M216T, and Q258A; or
r. the amino acids 121K, 126H, 204T, 212G, 216T, and 258N, preferably the substitutions P121K, N126H, D204T, S212G, M216T, and Q258N; or
s. a set of amino acid substitutions specified in Table 1.

In an embodiment the variant polypeptide has asparagine or threonine at the position 204.

In an embodiment the variant polypeptide further comprises the amino acids 121K, 126H, 216T, and 258N/A; preferably the amino acid substitutions P121K, N126H, M216T, and Q258N/A.

In an embodiment the variant polypeptide further comprises the amino acids 75C/V, 114T, 137E, 141T, 142D, 146R, 157G, 204C, 211W, 253Q, 267R, and 341P; or the amino acids D35Y, G70E, S80P, T161P, N176P, L179F, K180N, S187P, K276M, T277A, E315G, and A380P.

In an embodiment the variant polypeptide has an increased IP4 degradation activity, and/or a ratio of IP4 to IP6 activity of higher than 1.3, compared to the SEQ ID NO: 1 and/or SEQ ID NO: 2. It is thus possible to use substitutions in the presently claimed positions to change the enzyme activity of the variant polypeptide such that it has more activity towards IP4 substrates. Additionally, the present variant polypeptide may have IP6 degrading activity.

The increased relative IP4 degrading activity means higher specific IP4 degrading activity (more IP4 activity per mg phytase protein), a higher concentration of IP4 degrading phytase in spent culture medium after cultivation, a more stable protein having IP4 degrading activity, or any combinations of these. The increased relative IP4 degrading activity of the present variant polypeptide can thus be used to an advantage for example in compositions that are used to degrade inositol phosphates, and in particular where degradation of inositol phosphate to lower inositol phosphates is desired. A corresponding explanation is herein given to the relative IP6 degrading activity.

According to a second aspect is provided a recombinant host cell comprising genetic elements that allow producing at least one variant polypeptide, wherein the host cell is preferably selected from the group consisting of:
a. filamentous fungal cells from Division Ascomycota, Subdivision Pezizomycotina; preferably from the group consisting of members of the Class Sordariomycetes or Eurotiomycetes, Subclass Hypocreomycetidae or Sordariomycetidae or Eurotiomycetidae, Orders Hypocreales or Sordariales or Eurotiales, Families Hypocreacea or Nectriacea or Chaetomiaceae or Aspergillaceae, Genera *Trichoderma* (anamorph of *Hypocrea*) or *Fusarium* or *Acremonium* or *Humicola* or *Thermothelomyces* or *Myceliophthora* or *Aspergillus;*
   more preferably from the group consisting of species *Trichoderma reesei* (*Hypocrea jecorina*), *T. citrinoviridae, T. longibrachiatum, T. virens, T. harzianum, T. asperellum, T. atroviridae, T. parareesei, Fusarium oxysporum, F. gramineanum, F. pseudograminearum, F. venenatum, Acremonium (Cephalosporium) chrysogenum, Humicola insolens, H. grisea, Thermothelomyces thermophilus, Myceliophthora thermophila, Aspergillus niger, A. niger* var. *awamori* and *A. oryzae;*
b. bacterial cells, preferably gram-positive Bacilli such as *B*. *subtilis, B*. *licheniformis, B*. *megaterium, B*. *amyloliquefaciens, B*. *pumilus,* gram negative bacteria such as *Escherichia coli,* actinomycetales such as *Streptomyces sp*.; and
c. yeasts, such as *Saccharomyces cerevisiae, Schizosaccharomyces pombe, Pichia pastoris, Yarrowia lipolytica;* and
   more preferably the host cell is selected from filamentous fungal cells such as *Trichoderma,* or from gram-positive Bacilli such as *Bacillus;*
most preferably from *Trichoderma reesei* or from *Bacillus subtilis* or *B*. *pumilus* or *B*. *licheniformis* or *B*. *amyloliquefaciens.*

According to a third aspect is provided a recombinant host cell comprising genetic elements configured to produce at least one variant polypeptide of the present disclosure, and wherein the host cell is a transgenic plant cell.

According to an alternative third aspect is provided an enzyme composition comprising the present variant polypeptide.

According to a fourth aspect is provided a use of the present variant polypeptide or the present enzyme composition in the manufacturing of feedstuff or foodstuff feed additive, a dietary supplement, or a pharmaceutical.

According to a fifth aspect is provided a method of manufacturing the present variant polypeptide comprising:
a. providing a polynucleotide comprising genetic elements arranged to produce the present variant; and
b. expressing the polynucleotide in a recombinant host cell, preferably in the present recombinant host cell.

According to a sixth aspect is provided an animal feed comprising the present variant polypeptide, or the present enzyme composition, and at least one protein source of plant origin, and
a. optionally at least one further enzyme selected from protease, amylase, phytase, xylanase, endoglucanase, beta-glucanase, mannanase, cellulase, or a combination thereof; and
b. optionally at least one filler selected from maltodextrin, flour, salt, sodium chloride, sulphate, sodium sulphate, or a combination thereof.

According to a seventh aspect is provided a feed supplement comprising the present variant polypeptide or the present enzyme composition; and
a. optionally at least one further enzyme selected from protease, amylase, phytase, xylanase, endoglucanase, beta-glucanase, mannanase, cellulase, or a combination thereof; and
b. optionally at least one carrier or ingredient selected from maltodextrin, flour, salt, sodium chloride, sulphate, sodium sulphate, minerals, amino acids, prebiotics, probiotics, vitamins, or a combination thereof.

According to an eighth aspect is provided a method of degrading or modifying material containing phytic acid and/or phytate, comprising treating said material with an effective amount of the present variant polypeptide or the present enzyme composition.

### BRIEF DESCRIPTION OF THE FIGURES

Some example embodiments will be described with reference to the accompanying figures, in which:

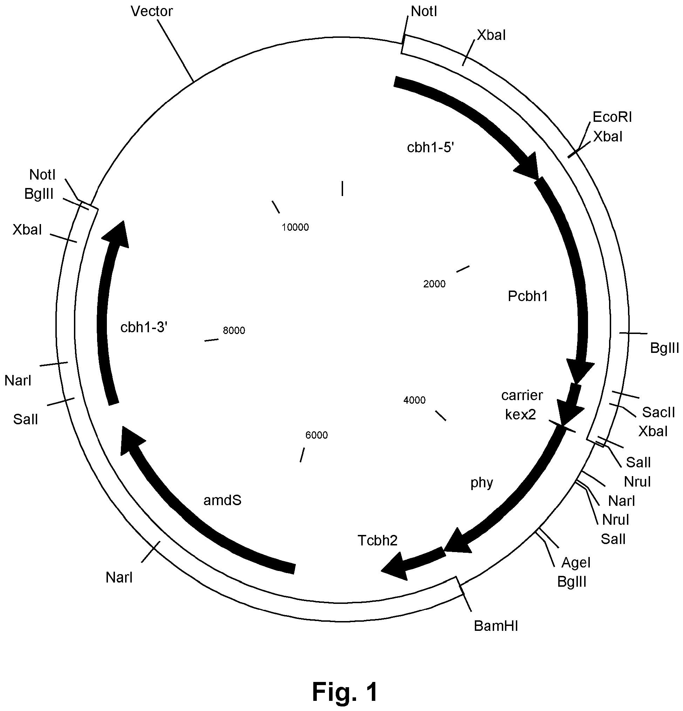

**Figure 1** shows a schematic picture of the expression plasmid used in the transformation of *Trichoderma reesei* for expression of parent phytase (SEQ ID NO:1) and phytase variant genes (phy). The expression of the recombinant genes in the host cell was controlled by use of the following genetic elements: *T. reesei cbh1* promoter (Pcbh1) for transcription initiation, and *T. reesei cbh2* (Tcbh2) terminator for transcription termination. *T. reesei cbh2* carrier encoding the CBHII CBM and linker region (carrier) was used instead of the native phytase signal sequence and Kex2 protease cleavage site (*kex2*) was included between the encoded carrier polypeptide and phytase. A synthetic gene (*amdS*) encoding the AmdS marker was included for selection of the transformants and *T. reesei cbh1* 3'- and 5'-flanking regions (cbh1-3' and cbh1-5', respectively) were used to optionally target the expression cassette to *cbh1* locus. The vector part (Vector) derives from pUC19. Picture was generated using Clone Manager Professional 9 from Sci-Ed Software. A selection of restriction enzyme sites is shown in the picture.
**Figure 2** outlines the in vitro test (GIT) outcome for selected phytase variants improved in inositol phosphate degradation. The residual amount of the sum of IP6+IP5+IP4 after corn-soybean meal treatment with the tested phytase is compared to the residual amount of these inositolphosphates without any phytase (blank) or the same activity of phytase SEQ ID NO:2 added. The dosing of the phytases was 250 FTU and 500 FTU per kg corn-soybean meal mix, respectively.
**Fig 2A** demonstrates a slightly further degradation of the sum of IP6 to IP4 compared to SEQ ID NO:2 phytase by variant 01107 after in vitro treatment (GIT) of a corn-soybean meal mix when added with 250 FTU/kg and 500 FTU/kg.
**Fig 2B** demonstrates a further degradation of the sum of IP6 to IP4 compared to SEQ ID No:2 phytase by variant 01134 after in vitro treatment (GIT) of a corn-soybean meal mix when added with 250 FTU/kg and 500 FTU/kg.
**Fig 2C** demonstrates a further degradation of the sum of IP6 to IP4 compared to SEQ ID No:2 phytase by variant 01135 after in vitro treatment (GIT) of a corn-soybean meal mix when added with 250 FTU/kg and 500 FTU/kg,
**Fig 2D** demonstrates a further degradation of the sum of IP6 to IP4 compared to SEQ ID No:2 phytase by variant 01135 after in vitro treatment (GIT) of a corn-soybean meal mix when added with 250 FTU/kg and 500 FTU/kg.

### SEQUENCE LISTING

SEQ ID NO: 1 Amino acid sequence of parent phytase without signal peptide having the following two amino acid substitutions compared to SEQ ID NO: 2: 211V and 253Y
SEQ ID NO: 2 Amino acid sequence of parent phytase without signal peptide

### DETAILED DESCRIPTION

As used herein, the term "phytase" means an enzyme having capability to enzymatically degrade phytic acid to lower inositol phosphates.

Phytases are classified into 3-, 5- or 6-phytases (EC 3.1.3.8, EC 3.1.3.72, and EC 3.1.3.26, respectively) based on the carbon position on the inositol ring at which they preferably initiate phosphate hydrolysis. 6-phytases preferably first remove the phosphate group at the C6 position.

The present invention also relates to a polynucleotide comprising a nucleic acid sequence encoding a phytase variant polypeptide according to the first aspect.

In an embodiment the polypeptide comprising the phytase variant comprises at least one further amino acid sequence selected from a signal sequence, a secretory sequence, a carrier polypeptide, binding domain, a tag, a linker, an enzyme, or any combination thereof.

The terms "phytase variant", "variant of phytase", or "variant polypeptide" mean a phytase molecule obtained by site-directed or random mutagenesis, insertion, substitution, deletion, recombination and/or any other protein engineering method, and which leads into a genetically modified phytase that differs in its amino acid sequence from the parent phytase such as a wild type phytase. The terms "wild type phytase ", "wild type enzyme", "wild type", or "wt" in accordance with the disclosure, describe a phytase enzyme with an amino acid sequence found in nature or a fragment thereof. The variant encoding gene can be synthesised or the parent gene be modified using genetic methods, e.g., by site-directed mutagenesis, a technique in which one or more than one mutation are introduced at one or more defined sites in a polynucleotide encoding the parent polypeptide. The term variant phytase may also be referred to by using a name given to the variant in the examples and in the tables.

As used herein, the term "mature polypeptide" means any polypeptide wherein at least one signal sequence or signal peptide or signal peptide and a putative pro-peptide, or a carrier peptide or a fusion partner is cleaved off. As used herein, a "peptide" and a "polypeptide" are amino acid sequences including a plurality of consecutive polymerized amino acid residues. For purpose of this disclosure, peptides are molecules including up to 20 amino acid residues, and polypeptides include more than 20 amino acid residues. The peptide or polypeptide may include modified amino acid residues, naturally occurring amino acid residues not encoded by a codon, and non-naturally occurring amino acid residues. As used herein, a "protein" may refer to a peptide or a polypeptide of any size. A protein may be an enzyme, a protein, an antibody, a membrane protein, a peptide hormone, regulator, or any other protein.

As used herein, "sequence identity" means the percentage of exact matches of amino acid residues between two optimally aligned sequences over the number of positions where there are residues present in both sequences. When one sequence has a residue with no corresponding residue in the other sequence, the alignment program allows a gap in the alignment, and that position is not counted in the denominator of the identity calculation.

As used herein, "sequence alignment" of the amino acid sequences means, aligning the sequences using Clustal Omega (1.2.4) multiple sequence alignment program (https://www.ebi.ac.uk/Tools/msa/clustalo/) as described by Sievers et al. 2011, and using the default settings.

Unless otherwise specified, all references to a certain amino acid position refer to an amino acid of the SEQ ID NO: 1 in said position, or to an amino acid present or missing in the corresponding position of an amino acid sequence aligned with SEQ ID NO: 1.

As used herein, the term "disulfide bridge", or "disulfide bond", or "SS bridge" refers to a bond formed between the sulfur atoms of cysteine residues in a polypeptide or a protein. Disulfide bridges can be naturally occurring, or non-naturally occurring, and, for example, introduced by way of amino acid substitution(s).

As used herein, the term "corresponding positions" or "corresponding amino acid position" means aligning at least two amino acid sequences according to identified regions of similarity or identity as pairwise alignment or as multiple sequence alignment, thereby pairing up the corresponding amino acids. In the present disclosure corresponding positions typically refers to a position corresponding to the position in SEQ ID NO: 1.

As used herein, "amino acid substitution" means an amino acid residue replacement with an amino acid residue that is different than the original amino acid in that specific replacement position. The term "amino acid substitution" can refer to conservative amino acid substitutions and non-conservative amino acid substitutions, which means the amino acid residue is replaced with an amino acid residue having a similar side chain (conservative), or a different side chain (non-conservative), as the original amino acid residue in that place.

The term "functional fragment" means a fragment or portion of the current variant, which retains about the same enzymatic function or effect.

The term "secretory signal sequence" or "signal sequence" or a "secretory peptide" refers to an amino acid sequence which is a component or a part of a larger polypeptide, and which directs the larger polypeptide through a secretory pathway of a host cell in which it is produced. The secretory signal sequence can be native or it can be obtained from another source. Depending on the host cell, the larger polypeptide may be cleaved from the secretory peptide during transit through the secretory pathway, thereby forming a mature polypeptide lacking the secretory peptide.

The term carrier polypeptide or fusion partner refers to a polypeptide into which the protein of interest (phytase) is translationally fused to improve the yield. The carrier/fusion partner can be either homologous or heterologous to production host in its origin and can be a full-length protein or a fragment of a protein (e.g., a core, a CBM or a CBM and linker region). It is preferably encoded by a gene or a nucleotide sequence with good expression level.

"Phytase activity" as used herein, refers to the phytic acid degrading activity. Examples 3 and 4 provide examples of a method for determining phytase activity. Accordingly, IP4 activity refers to the capability to degrade IP4, and IP6 activity refers to the capability to degrade IP6. The ratio of IP4 degrading activity to IP6 degrading activity is sometimes disclosed herein in as IP4/IP6 compared to the IP4/IP6 ratio of a phytase having the SEQ ID NO: 2. IP4 degrading activity and IP6 degrading activity can be determined as described in the Examples.

In an embodiment the term "enzyme composition" means an enzymatic fermentation product, possibly isolated and purified, typically produced by a pure culture of a microorganism. The enzyme composition usually comprises a number of different enzymatic activities produced by the microorganism. In another embodiment the enzyme composition is a mixture of monocomponent enzymes, preferably enzymes derived from bacterial or fungal species by using conventional recombinant production techniques. The enzyme composition may contain for example stabilators and preservatives which prevent microbial growth and improve storage stability.

As used herein, a "host cell" means any cell type that is susceptible to transformation, transfection, transduction, mating, crossing, CRISPR-Cas, or the like with a nucleic acid construct or expression vector comprising a polynucleotide. The term "host cell" encompasses any progeny that is not identical due to mutations that occur during replication. Non-limiting examples of a host cell are fungal cells, preferably a filamentous fungal cell (e.g., *Trichoderma* or *Trichoderma reesei, Aspergillus* or *Aspergillus oryzae* or *A. niger, Thermothelomyces or Thermothelomyces heterothallica, Myceliophthora or Myceliophthora thermophila,* or *Humicola or Humicola insolens* or *Fusarium* or *Fusarium venenatum),* bacterial cells, preferably gram-positive Bacilli (e.g., *Bacillus subtilis, B*. *licheniformis, B*. *megaterium, B*. *amyloliquefaciens, B*. *pumilus),* gram-negative bacteria (e.g., *Escherichia coli),* actinomycetales (e.g., *Streptomyces sp., Nonomuraea flexuosa*) and yeasts (e.g., *Saccharomyces cerevisiae, Schizosaccharomyces pombe, Pichia pastoris, Yarrowia lipolytica).*

In another embodiment the phytase variant is obtained by recombinant production in plant cells, i.e., in a transgenic plant.

The recombinant host cell can be used to produce the phytase variant and to contain a polynucleotide encoding it. The recombinant host cell can be operably linked to one or more control sequences that direct the production of the variant, and that make it possible to initiate the production of the present phytase variant by a stimulus, as is known in the field. The recombinant host cell is useful also in preparation of variants with different properties. For example, a host cell can be selected, which provides post-translational modifications beneficial for stability or activity, or which facilitates post-processing of a variant produced in the host cell.

In an embodiment the host cell is non-pathogenic. This is particularly advantageous for using the host cell or the phytase variant produced in it for animal feed.

In an embodiment the composition containing the phytase variant is food or feed, and it may further comprise plant material which contains phytic acid.

In an embodiment the composition is a food additive or a feed additive further comprising at least one of: at least one trace mineral, at least one amino acid, in particular lysine, water soluble vitamin, fat soluble vitamin, prebiotic, and probiotic.

In an embodiment the composition is a food additive or a feed additive complying with the requirements of Regulation (EC) No 1831/2003 of the European Parliament and of the Council of 22 September 2003 on additives for use in animal nutrition.

In an embodiment the composition is in a form of a liquid composition or a solid composition such as solution, dispersion, paste, pellet, powder, granule, coated granule, tablet, cake, crystal, crystal slurry, gel, extrude, precipitate, premix, or a combination thereof.

The term "promoter" refers to a portion of a gene containing DNA sequence(s) that provide for the binding of RNA polymerase and initiation of transcription. Promoter sequences are commonly, but not always, found in the 5' non-coding regions of genes.

The term "propeptide" or "pro-peptide" is a part of a protein that is cleaved during maturation or activation. Once cleaved, a propeptide generally has no independent biological function.

As used herein, the terms "domain" and "region" can be used interchangeably with the term "module".

The following abbreviations are used for amino acids:

| | | |
|---|---|---|
| A | Ala | Alanine |
| C | Cys | Cysteine |
| D | Asp | Aspartic acid |
| E | Glu | Glutamic acid |
| F | Phe | Phenylalanine |
| G | Gly | Glycine |
| H | His | Histidine |
| I | Ile | Isoleucine |
| K | Lys | Lysine |
| L | Leu | Leucine |
| M | Met | Methionine |
| N | Asn | Asparagine |
| P | Pro | Proline |
| Q | Gln | Glutamine |
| R | Arg | Arginine |
| S | Ser | Serine |
| T | Thr | Threonine |
| V | Val | Valine |
| W | Trp | Tryptophan |
| Y | Tyr | Tyrosine |
| X | Xaa | Any one of the above amino acids |

Substitutions are described herein by using of the following nomenclature: amino acid residue in the protein scaffold, i.e., the parent sequence; position; substituted amino acid residue(s). According to this nomenclature the substitution of, for instance, a single residue of alanine to tyrosine residue at position 23 is indicated as Ala23Tyr or A23Y. A substitution of any amino acid in position 23 to tyrosine is indicated as Xaa23Tyr or X23Y or 23Y. A substitution of a tyrosine in position 179 to phenylalanine, tryptophan, or leucine is indicated as Y179F/W/L.

As used herein, the term "comprising" includes the broader meanings of "including", "containing", and "comprehending", as well as the narrower expressions "consisting of" and "consisting only of".

As used herein, the term "expression" includes any step or all steps involved in the production of a polypeptide in a host cell including, but not limited to, transcription, translation, post-translational modification, and secretion. Expression may be followed by harvesting, i.e., recovering, the host cells or the expressed product.

In an embodiment the phytase variant has phytase activity, and an amino acid sequence with at least 80 %, at least 85 %, at least 90 %, at least 92%, at least 94%, at least 95 %, at least 96%, at least 97%, at least 98%, or at least 99% sequence identity with amino acids of SEQ ID NO: 1. In an embodiment the variant polypeptide does not have 100% sequence identity with amino acids of SEQ ID NO: 1. In an embodiment, the amino acid numbering of the variant polypeptide corresponds to that of SEQ ID NO: 1. In an alternative embodiment, the amino acid numbering of the variant polypeptide corresponds to that of SEQ ID NO: 1 partially.

In an embodiment the total number of the amino acid substitutions in the variant polypeptide, compared to the SEQ ID NO: 1, is at least 2. The at least one further amino acid may be selected from the positions disclosed herein. In another embodiment the total number of substitutions is at least 5, at least 10, at least 15, at least 20 or at least 25; or 5, 10, 15, 20, or 25. In an embodiment the substitution or the substitutions are made in the non-conservative region of the amino acid sequence. In another embodiment the variant polypeptide comprises the substitutions specified in any claim, and additional substitutions in non-conserved region of the amino acid-sequence. The effect these additional substitutions have on the properties can be analyzed as described in the Examples.

In an embodiment the variant polypeptide, or the functional fragment, has a predicted molecular weight between 40 and 60 kDa, preferably between 43-55 kDa. The predicted molecular weight can be determined by calculating the sum of the molecular weights of the individual amino acids in the variant polypeptide, or in its functional fragment. When the predicted molecular weight of the variant polypeptide is within the above range, the structure of the variant polypeptide may be similar with the parent sequence to which the substitutions are made.

In an embodiment the composition is provided in the form of a liquid composition or a solid composition, such as solution, dispersion, paste, powder, granule, granulate, coated granulate, tablet, cake, crystal, crystal slurry, gel, or pellet.

In an embodiment the phytic acid is degraded in a plant-based material or partly plant based material which contains phytic acid.

In an embodiment the present phytase variant is used in an animal feed, and the animal is a ruminant or a non-ruminant. In another embodiment the animal is a cattle like beef or cow, a sheep or goat. In another embodiment the non-ruminant include poultry (such as broiler, layer and turkey and duck); pigs (such as piglets, growing pigs and sows); fish (such as salmonids, carp, tilapia and catfish) and crustaceans. In an embodiment the feed is animal feed intended to be fed to animals such as any compound feed or mixture. In another embodiment feed comprises or consists of grains such as maize, wheat, oats, barley, sorghum and rice; protein sources like soybean meal, sunflower meal and canola meal as well as of minerals. The feed, wherein the present variant is used, has improved nutritional value compared to a feed without the variant. The present composition and the present phytase variant degrade phytic acid of the feed and thereby increase its nutritional value for the animals. The animal feed, wherein the present phytase variant or the present composition is used, can be formulated in the form of a wet composition or a dry composition.

### EXAMPLES

The present invention is further disclosed by the following non-limiting examples.

### Example 1. Computational design of variants with improved IP4 activity

### Computational design of variants with improved IP4 activity

Molecular structures of the most probable IP4 intermediates of 6-phytases according to Ariza *et al.* 2013 (doi:10.1371/journal.pone.0065062) were constructed, which are IP4 with a phosphate at positions 1, 2, 3 and 4 (IP41,2,3,4), IP4 with a phosphate at positions 1, 2, 5 and 6 (IP41,2,5,6) and IP4 with a phosphate at positions 1, 2, 4 and 6 (IP41,2,4,6). All atoms not part of the protein chain in the structural model of 6-phytase were removed and hydrogens were added according to a pH of 5.0. For each of the prepared IP4 intermediates a solvated and neutralized system was constructed by adding the respective IP4 intermediate near the substrate cavity of the enzyme and filling the simulation box with water and ions. Each thus constructed system was then equilibrated at room temperature and a pressure of 1 bar as described above. Enzyme-substrate interactions were sampled from the equilibrated systems with an accelerated molecular dynamics approach. For each of the thus generated simulation trajectories a distribution of productive and unproductive enzyme-substrate conformations was derived based on first principle knowledge of the phytase reaction mechanism. All positions in the substrate cavity were analyzed by geometrical criteria to derive candidates for amino acid substitutions that potentially reduce unproductive binding conformations and potentially enhance productive binding conformations (substitution candidates). For each of the thus derived substitution candidates, a system was constructed, equilibrated, simulated and analyzed as described above to yield distributions of productive and unproductive binding conformations. Substitution candidates were ranked by their improvement in the ratio of productive conformations over unproductive conformations and the most promising candidates were selected for experimental characterization. The designed phytase variants are described in detail in Table 1.

**Table 1. List of phytase variants designed in the computational design and simulation, and selected for production and characterisation. The amino acid numbering corresponds to the amino acid numbering of the mature parent phytase molecule presented in SEQ ID NO: 1.**

| **Variant code** | **Mutation(s)** |
|---|---|
| 01085 | N126H |
| 01090 | N126H, D204N |
| 01091 | N126H, D204T |
| 01092 | N126H, S212G |
| 01093 | N126H, Q258A |
| 01094 | N126H, Q258N |
| 01102 | P121K, N126H, M216T |
| 01107 | N126H, D204N, S212G |
| 01108 | N126H, D204N, Q258A |
| 01109 | N126H, D204N, Q258N |
| 01110 | N126H, D204T, S212G |
| 01111 | N126H, D204T, Q258A |
| 01112 | N126H, D204T, Q258N |
| 01113 | N126H, S212G, Q258A |
| 01114 | N126H, S212G, Q258N |
| 01119 | P121K, N126H, D204N, M216T |
| 01120 | P121K, N126H, D204T, M216T |
| 01121 | P121K, N126H, S212G, M216T |
| 01122 | P121K, N126H, M216T, Q258A |
| 01123 | P121K, N126H, M216T, Q258N |
| 01131 | N126H, D204N, S212G, Q258A |
| 01132 | N126H, D204N, S212G, Q258N |
| 01133 | N126H, D204T, S212G, Q258A |
| 01134 | N126H, D204T, S212G, Q258N |
| 01135 | P121K, N126H, D204N, S212G, M216T |
| 01136 | P121K, N126H, D204N, M216T, Q258A |
| 01137 | P121K, N126H, D204N, M216T, Q258N |
| 01138 | P121K, N126H, D204T, S212G, M216T |
| 01139 | P121K, N126H, D204T, M216T, Q258A |
| 01140 | P121K, N126H, D204T, M216T, Q258N |
| 01141 | P121K, N126H, S212G, M216T, Q258A |
| 01142 | P121K, N126H, S212G, M216T, Q258N |
| 01147 | P121K, N126H, D204N, S212G, M216T, Q258A |
| 01148 | P121K, N126H, D204N, S212G, M216T, Q258N |
| 01149 | P121K, N126H, D204T, S212G, M216T, Q258A |
| 01150 | P121K, N126H, D204T, S212G, M216T, Q258N |

### Example 2. Production of phytase variants

Standard molecular biology methods were used in the isolation and enzyme treatments of DNA (e.g., isolation of plasmid DNA, digestion of DNA to produce DNA fragments), in *E. coli* transformations, sequencing etc. The basic laboratory methods used were either as described by the enzyme, reagent or kit manufacturer or as described in the standard molecular biology handbooks, e.g., Sambrook and Russell (2001) or as described in the following examples.

The phytase genes encoding the designed phytase variants (Example 1, Table 1) were ordered as synthetic genes using codons optimised for preparation of transformants and for expression in *Trichoderma reesei.*

The phytases in the constructions were expressed from *T. reesei cbh1* (*cel7A*) promoter using a carrier polypeptide (CBM and linker) encoding sequence from *T. reesei cbh2* (*cel6A*)*.* A Kex2 protease cleavage site was included between the carrier polypeptide and phytase like described in Paloheimo *et al.* 2003. The transcription was terminated using *cbh2* terminator, followed in the construction by a synthetic amdS marker gene. In addition, the constructions contain *cbh1* 3' and 5' flanking regions for optionally targeting the expression vector into the *cbh1* locus (Figure 1).

Circular expression plasmids were transformed in *T*. reesei protoplasts. The transformants were selected on plates containing acetamide as the sole nitrogen source. The host strain used lacks the four major endogenous *T*. *reesei* cellulases: CBHI/Cel7A, CBHII/Cel6A, EGI/Cel7B and EGII/Cel5A. The transformations were performed according to Penttilä et al., 1987, with the modifications described in Karhunen et al., 1993. Alternatively, CRISPR-Cas technology can be used in transformations.

The transformants were sporulated on potato dextrose agar (PDA) prior to cultivation.

The transformants were cultivated on 96-well plates (Havukainen et al., 2020) to analyse the phytase production of the transformants. Phytase activity of the recombinant variant phytases was measured from the culture supernatants as release of inorganic phosphate from sodium phytate as described in Example 3. Production of the recombinant protein was also detected from the culture supernatant by sodium dodecyl sulphate polyacrylamide gel electrophoresis (SDS-PAGE).

All the transformants produced phytase activity. Transformants producing variant phytases with the highest phytase activity and the reference strain producing the parent phytase were purified on selection plates through single conidia prior to sporulating them on PDA. The purified, selected transformants were cultivated in shake flasks and/or bioreactors in complex cellulase-inducing medium to obtain material for further characterisation and application tests.

### Example 3. Phytase activity assays

Phytase activity was analysed from culture supernatants using IP6 and isolated IP4 form as a substrate as described below.

The phytic acid dodecasodium salt (IP6) used in the analysis was purchased from LabChem (C₆H₆Na₁₂O₂₄P₆, EE05501). Method to generate mainly IP4 specific isomer fraction was performed in 4 steps. In a first step Quantum Blue phytase was immobilized on 5ml HiTrap NHS-activated sepharose column from General Electric (Boston, USA) as described in Greiner & Konietzky, (1996). In a second step, the immobilized phytase degraded IP6 in 0.1 M Na-acetate buffer pH 5.0 stepwise to lower inositol phosphates. The flowrate of 5ml/min achieved the highest portion of IP4. The next step was used to remove phosphate from the solution and separate IP4 from other undesired inositol phosphates. Therefore, a manually packed anion exchange column with AG1-x4 resin from Bio-Rad (Hercules, USA) was loaded with the produced inositol phosphate mix and IP₄ was eluted with 0.5 M HCl. In the last step, HCl was removed with a rotating evaporator and IP4 re-dissolved in water.

The activity of samples from the microtiter plate cultivations (Example 2) was screened using Fluent^{®} automation workstation (Tecan Group Ltd, Männedorf, Switzerland) as follows. The samples used in the assay are diluted in a 0.2 M citrate buffer (pH 4.0) containing 0.01 % Tween 20 (Merck 822184). 0.01 % of Tween 20 is added also to the substrate solution. The substrate concentration used in this analysis is 6.35 mM. 200 µl of sample dilution and 200 µl of substrate are mixed and incubated at 37 °C. After exactly 15 min incubation 400 µl of 15 % (w/v) TCA solution (Trichloroacetic acid, CCl₃COOH, Merck 807) is added to the mixture to stop the reaction. 25 µl of reaction mixture is transferred into another well and 225 µl of water is added to make 1:10 dilution. 250 µl of colour reagent consisting of three volumes of 1 M sulphuric acid (H₂SO₄, Merck 731), one volume of 2.5 % (w/v) ammonium molybdate ((NH₄)₆Mo₇O₂₄ · 4 H₂O, Merck 1182) and one volume of 10 % (w/v) ascorbic acid (C₆H₈O₆, AnalaR Normapur 20150) is added and the colour reaction is incubated for 20 min at 50 °C. After the incubation the absorption is measured at 820 nm. The absorbance of the sample is compared to that of a reference sample which is the SEQ ID NO: 2.

The activity from the shake flask and fermentation cultivations (Example 2) was analysed using a phytase activity assay (PPU). In PPU analysis one activity unit is the quantity of enzyme that liberates 1 µmol of inorganic phosphate per one minute from sodium phytate at pH 4.0 and at 37 °C in a 15 min reaction time. The substrate concentration used in this analysis is 12.7 mM

The samples used in the assay are diluted in a reaction buffer (0.2 M citrate buffer, pH 4.0) and 1 ml of enzyme solution is used in the analysis. 1 ml of substrate is added to the enzyme solution and after incubating the mixture at 37°C for exactly 15 min, the reaction is stopped by adding 2 ml of 15 % (w/v) TCA solution (Trichloroacetic acid, , Merck 807). The reaction mixture is cooled to room temperature and after this 1:10 dilution is done by mixing 0.2 ml of the mixture and 1.8 ml of water in a test tube. 2.0 ml of freshly made colour reagent is added to the tube and mixed. The colour reagent consists of three volumes of 1 M sulphuric acid (H₂SO₄, Merck 731), one volume of 2.5 % (w/v) ammonium molybdate ((NH₄)₆Mo₇O₂₄ · 4 H₂O, Merck 1182) and one volume of 10 % (w/v) ascorbic acid (C₆H₈O₆, AnalaR Normapur 20150). The tubes are incubated at 50 °C for 20 min and cooled to room temperature. After this the absorption is measured at 820 nm against the enzyme blank. For the enzyme blank the substrate is added after the TCA and the 15 min incubation is passed. The amount of liberated phosphate is determined via a standard curve of the color reaction with a phosphate solution of known concentration.

The activity for the samples used in gastrointestinal tract test (GIT) (Example 4) was analysed by an internal validated phytase method (FTU assay). In FTU assay inorganic phosphate released from sodium phytate substrate by the hydrolytic enzymatic action of phytase is detected. Colour formation, which is measured spectrophotometrically, is the result of molybdate and vanadate ions complexing with inorganic phosphate. One phytase unit (FTU) is the quantity of enzyme that liberates 1 µmol of inorganic phosphate per minute from sodium phytate at 37 °C, pH 5.50, using 60 min incubation time.

In the assay, 2.0 ml of 1 % sodium phytate substrate (LabChem EE05501, in 250 mM sodium acetate buffer, pH 5.5 and including 1 mM CaCl₂·2H₂O and 0.01% Tween 20) is pipetted to plastic centrifuge tubes. The substrate tubes are pre-incubated for 5 -10 minutes at 37 °C after which 1.0 ml of diluted enzyme sample is added. After exactly 60 min incubation 2.0 ml of colour stop solution is added and tube contents are mixed by vortexing. Enzyme blanks are prepared like the sample but the colour stop solution is added to the substrate tubes prior to addition of the diluted enzyme sample. For colour reaction the tubes are incubated for 20 min at room temperature after which they are centrifuged at 4000 rpm for 10 minutes. The sample absorbance is measured against an enzyme blank at 415 nm. For the activity units, a potassium phosphate standard curve (pH 5.50) is prepared (dried KH₂PO₄, Merck 1.04873.1 is used for the standard; drying at 105 °C for 2 hours before weighting).

The stop solution is prepared as follows (preparation just prior to use): for 100 ml of colour stop solution, 25 ml of stock ammonium heptamolybdate (20 g of (NH₄)₆Mo₇O₂₄ · 4H₂O, Merck 1182 in 180 ml of water, add 2 ml of ammonium hydroxide (NH₄OH, Sigma-Aldrich 221228 28-30 %), final volume 200 ml) is mixed with 25 ml of stock ammonium vanadate solution (0.47 g of ammonium vanadate (NH₄VO₃, Riedel de Haen 31153) in 160 ml of water; once the completely dissolved, 4 ml of 22.75 % nitric acid solution is added, final volume 200 ml). Then, 16.5 ml of 22.75 % nitric acid solution (HNO₃, Merck 1.00456) is added after which distilled water is added to make up the volume to 100 ml in volumetric flask.

**Table 2. IP6 and IP4 activities and the ratio of IP4 activity to IP6 activity of selected variants. The numbers presented here are relative activities compared to SEQ ID NO: 2. The results show that the variants have increased IP4 degradation activity, and/or an increased ratio of IP4 to IP6 activity compared to SEQ ID NO: 2.**

| **Variant code** | **IP6 activity** | **IP4 activity** | **IP4/IP6** |
|---|---|---|---|
| SEQ ID NO: 2 | 1.00 | 1.00 | 1.00 |
| 01085 | 1.34 | 1.46 | 1.09 |
| 01090 | 1.56 | 1.81 | 1.16 |
| 01091 | 1.15 | 1.71 | 1.48 |
| 01092 | 1.65 | 2.19 | 1.33 |
| 01093 | 1.27 | 1.45 | 1.14 |
| 01094 | 1.27 | 1.84 | 1.44 |
| 01102 | 1.10 | 1.60 | 1.46 |
| 01107 | 1.48 | 2.15 | 1.45 |
| 01108 | 1.06 | 1.44 | 1.36 |
| 01109 | 1.16 | 1.86 | 1.61 |
| 01110 | 1.12 | 1.72 | 1.53 |
| 01111 | 1.09 | 1.39 | 1.27 |
| 01112 | 1.08 | 1.55 | 1.44 |
| 01113 | 0.97 | 1.48 | 1.52 |
| 01114 | 1.43 | 1.95 | 1.37 |
| 01119 | 1.34 | 1.82 | 1.36 |
| 01120 | 0.95 | 1.46 | 1.53 |
| 01121 | 1.09 | 1.60 | 1.47 |
| 01122 | 0.70 | 1.02 | 1.44 |
| 01123 | 0.99 | 1.52 | 1.54 |
| 01131 | 0.99 | 1.50 | 1.53 |
| 01132 | 1.14 | 1.66 | 1.46 |
| 01133 | 0.90 | 1.23 | 1.37 |
| 01134 | 1.00 | 1.82 | 1.82 |
| 01135 | 1.00 | 1.77 | 1.78 |
| 01136 | 0.53 | 0.81 | 1.52 |
| 01137 | 0.86 | 1.31 | 1.52 |
| 01138 | 0.89 | 1.59 | 1.78 |
| 01139 | 0.64 | 1.00 | 1.56 |
| 01140 | 1.03 | 1.56 | 1.51 |
| 01141 | 0.61 | 1.06 | 1.74 |
| 01142 | 0.95 | 1.58 | 1.66 |
| 01147 | 0.59 | 1.00 | 1.69 |
| 01148 | 0.81 | 1.09 | 1.34 |
| 01149 | 0.64 | 1.13 | 1.76 |
| 01150 | 0.92 | 1.57 | 1.71 |

### Example 4. Gastrointestinal tract test (GIT) results

The comparison of selected novel phytase candidates in their ability to degrade phytate in feed materials was done using an in vitro gastrointestinal simulation test system (GIT) SEQ ID NO:2 was used as a reference.

Phytases to be tested are added with a defined activity level to a feed ingredient mix (60% corn; 40% soybean meal) following athree-step continuous in vitro test simulating the animal digestive conditions. The reactions are run at 40°C and at corresponding pHs and changes of pH as in the crop, gizzard and small intestine of broilers. Additionally, digestive proteases are added. To succeed in the GIT assay, the phytase needs to have a combination of beneficial biochemical properties. It needs to resist and act at different pHs at the temperature of the digestive tract while being resistant to proteases of the digestive tract. Details of the in vitro test system are described by Sommerfeld et al., 2017.

At the end of the in vitro test inositol phosphates are extracted and phytase removed from the supernatant before analysis of inositol phosphates (IP6 - IP3) using high performance liquid chromatography method (HPLC) according to Blaabjerg et al. 2010.

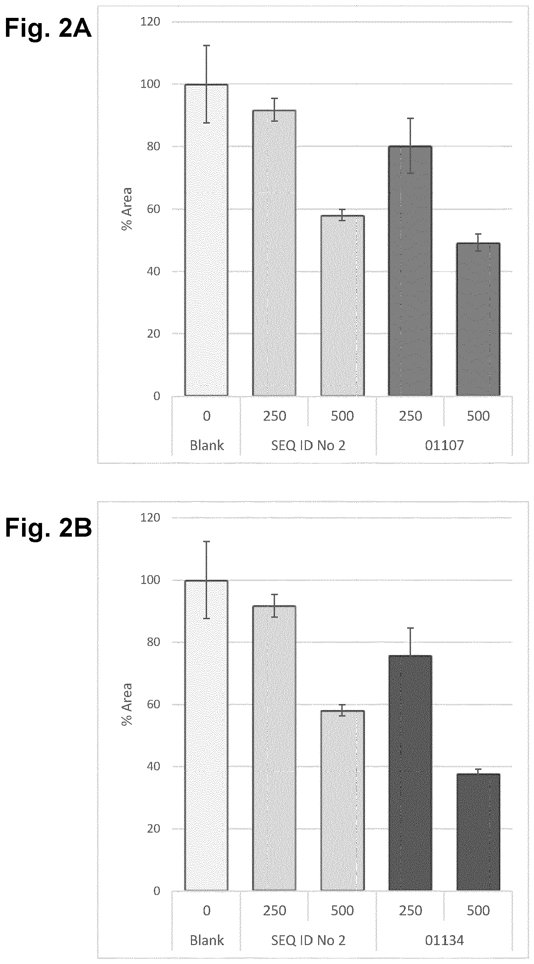

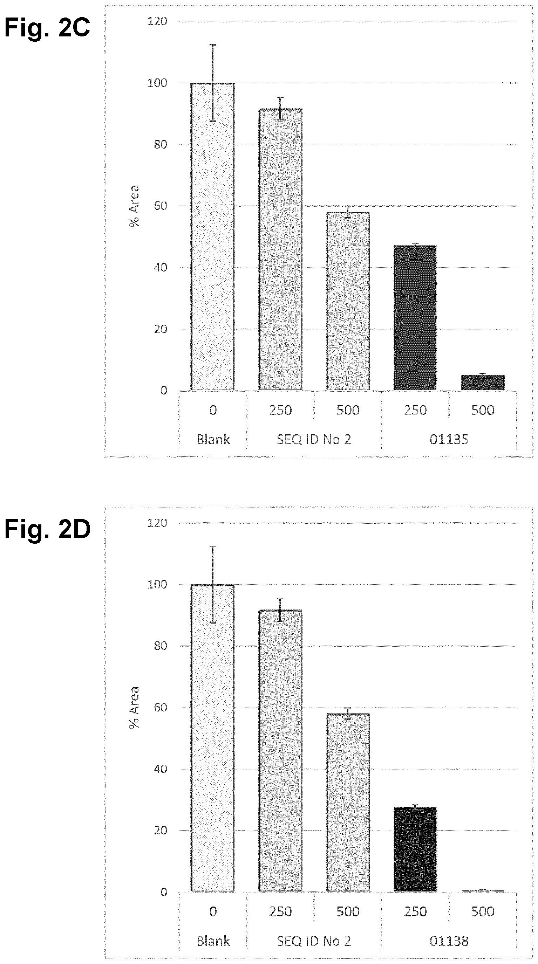

The results obtained from the GIT test are shown in Figure 2. Fig 2A to D demonstrate a further degradation of the sum of IP6 to IP4 compared to phytase SEQ ID NO:2 by phytase variants 01107 (Fig. 2 A), 01134 (Fig. 2 B), 01135 (Fig. 2 C) and 01138 (Fig. 2 D) after in vitro treatment (GIT) of a corn-soybean meal mix when added with 250 FTU/kg and 500 FTU/kg.

### REFERENCES

Ariza A, Moroz OV, Blagova EV, Turkenburg JP, Waterman J, Roberts SM, Vind J, Sjøholm C, Lassen SF, De Maria L, Glitsoe V, Skov LK and KS Wilson. 2013. Degradation of phytate by the 6-phytase from Hafnia alvei: a combined structural and solution study. PloS one 2013. 8(5), e65062. https://doi.org/10.1371/journal.pone.0065062
Bedford M.R. and C.L. Walk. 2016.Reduction of phytate to tetrakisphosphate (IP4) to trisphosphate (IP3), or perhaps even lower, does not remove its antinutritive properties. In: Phytate destruction - consequences for precision animal nutrition. Eds. Walk, C.L., Kühn, I., Stein, H.H., Kidd, M.T. and Rodehutscord, M. Wageningen 20 Academic publishers: 45-52.
Blaabjerg, K., H. Jørgensen, A. H. Tauson, and H. D. Poulsen. 2010. Heat-treatment, phytase and fermented liquid feeding affect the presence of inositol phosphates in ileal digesta and phosphorus digestibility in pigs fed a wheat and barley diet. Journal Article 4:876-885.
Greiner R. and U. Konietzny. 1996. Construction of a bioreactor to produce special breakdown products of phytate. Journal of Biotechnology, 48 (1-2): 153-159.
Havukainen S, Valkonen M, Koivuranta K and Landowski CP. 2020. Studies on sugar transporter CRT1 reveal new characteristics that are critical for cellulase induction in Trichoderma reesei. Biotechnol. Biofuels 2020 Sep 14;13:158. doi: 10.1186/s13068-020-01797-7. eCollection 2020.
Joutsjoki VV, TK Torkkeli and KMH Nevalainen. 1993. Transformation of Trichoderma reesei with the Hormoconis resinae glucoamylase P (gamP) gene: production of a heterologous glucoamylase by Trichoderma reesei. Curr. Genet. 24:223-228.
Karhunen T, A Mäntylä, KMH Nevalainen and PL Suominen. 1993. High frequency one-step gene replacement in Trichoderma reesei. I. Endoglucanase I overproduction. Mol. Gen. Genet. 241:515-522.
Lee, S. A., and M. R. Bedford. 2016. Inositol- an effective growth promotor? Worlds Poultry Science 72, doi:10.1017/S0043933916000660
Menezes-Blackburn, D.,S. Gabler and R. Greiner. 2015. Performance of seven commercial phytases in an in Vitro simulation of poultry digestive tract. J. Agric. Food Chem., 63:6242-6149
Paloheimo M, A Mäntylä, J Kallio, and P Suominen. 2003. High-yield production of a bacterial xylanase in the filamentous fungus Trichoderma reesei requires a carrier polypeptide with an intact domain structure. Appl. Env. Microbiol. 69:7073-7082.
Penttilä M, H Nevalainen, M Rättö, E Salminen and J Knowles. 1987. A versatile transformation system for the cellulolytic filamentous fungus Trichoderma reesei. Gene 61:155-164.
Sambrook J and DW Russell. 2001. Molecular cloning, a laboratory manual. Cold Spring Harbor Laboratory, New York, US.
Sievers F, A Wilm, D Dineen, TJ Gibson, K Karplus, W Li, R Lopez, H McWilliam, M Remmert, J Söding, JD Thompson and DG Higgins 2011. Fast, scalable generation of high-quality protein multiple sequence alignments using Clustal Omega. Mol. Syst. Biol. 7:539.
Sommerfeld M, Schollenberger, L Hemberle and M Rodehutscord 2017 Modification and application of an in vitro assay to examine inositol phosphate degradation in the digestive tract of poultry. Science of Food and Agiculture; 97 (12), p 4219-4226; doi.org/10.1002/jsfa.8297
Xu P, J Price, A Wise and PJ Aggett. 1992. Interaction of Inositol Phosphates with Calcium, Zinc, and Histidine. Journal of Inorganic Biochemistry 47: 119 - 130.
Zeller E, M Schollenberger, I Kühn and M Rodehutscord. 2015. Hydrolysis of phytate and formation of inositol phosphate isomers without or with supplemented 30 phytases in different segments of the digestive tract of broilers. Journal Nutritional Science 4, e1: 1 - 12.

The foregoing description has provided by way of non-limiting examples of particular implementations and embodiments a full and informative description of the best mode presently contemplated by the inventors for carrying out the invention.

It is however clear to a person skilled in the art that the invention is not restricted to details of the embodiments presented in the foregoing, but that it can be implemented in other embodiments using equivalent means or in different combinations of embodiments without deviating from the characteristics of the invention.

## Claims

1. A variant polypeptide comprising an amino acid sequence having at least 80% amino acid sequence identity with SEQ ID NO: 1, wherein the variant has:
an amino acid substitution at the position 126;
phytase activity; and
wherein the amino acid numbering corresponds to the amino acid numbering of the SEQ ID NO: 1.

2. The variant polypeptide of claim 1 having a histidine at the position 126.

3. The variant polypeptide of claim 1 or 2 comprising at least one further amino acid substitution at a position selected from 121, 204, 212, 216, and 258.

4. The variant polypeptide of claim 3, wherein the at least one further amino acid substitution results into the presence of at least one of the following amino acids: 121K, 204N/T, 212G, 216T, and 258N/A.

5. The variant polypeptide of any one of claims 3-4, wherein the at least one further amino acid substitution is a substitution selected from P121K, D204N/T, S212G, M216T, and Q258N/A.

6. The variant polypeptide of any one of claims 1-5, wherein the variant polypeptide is an *E*. *coli* 6-phytase.

7. The variant polypeptide of any one of claims 1-6, wherein the variant polypeptide comprises the amino acids:
a. 126H; and
b. at least one amino acid selected from: 121K, 204N/T, 212G, 216T, and 258N/A.

8. The variant polypeptide of any one of claims 1-7, wherein the variant polypeptide comprises the amino acid substitutions:
a. N126H; and
b. at least one amino acid substitution from: P121K, D204N/T, S212G, M216T, and Q258N/A.

9. The variant polypeptide of any one of claims 1-8, wherein the variant polypeptide comprises:
a. the amino acids 126H and 204N/T, preferably the substitutions N126H and D204N/T; or
b. the amino acids 126H and 212G, preferably the substitutions N126H and S212G; or
c. the amino acids 126H and 258A/N, preferably the substitutions N126H and Q258A/N; or
d. the amino acids 126H, 204N, and 212G, preferably the substitutions N126H, D204N, and S212G; or
e. the amino acids 126H, 204N, and 258N, preferably the substitutions N126H, D204N, and Q258N; or
f. the amino acids 126H, 204T, and 212G, preferably the substitutions N126H, D204T, and S212G; or
g. the amino acids 126H, 212G, and 258N, preferably the substitutions N126H, S212G, and Q258N; or
h. the amino acids 121K, 126H, and 216T, preferably the substitutions P121K, N126H, and M216T; or
i. the amino acids 126H, 204T, 212G, and 258N, preferably the substitutions N126H, D204T, S212G, and Q258N; or
j. the amino acids 121K, 126H, 204N, 212G, and 216T, preferably the substitutions P121K, N126H, S212G, and M216T.

10. The variant polypeptide of any one of claims 1-9, wherein the variant polypeptide comprises:
a. the amino acids 126H, 204N, 258N, preferably the substitutions N126H, D204N, and Q258N; or
b. the amino acids 126H, 204T, and 212G, preferably the substitutions N126H, D204T, and S212G; or
c. the amino acids 126H, 212G, and 258A, preferably the substitutions N126H, S212G, and Q258A; or
d. the amino acids 121K, 126H, 204T, and 216T, preferably the substitutions P121K, N126H, D204T, and M216T; or
e. the amino acids 121K, 126H, 216T, and 258N, preferably the substitutions P121K, N126H, M216T, and Q258N; or
f. the amino acids 126H, 204N, 212G, and 258A, preferably the substitutions N126H, D204N, S212G, and Q258A; or
g. the amino acids 126H, 204T, 212G, 258N, preferably the substitutions N126H, D204T, S212G, and Q258N; or
h. the amino acids 121K, 126H, 204N, 212G, and 216T, preferably the substitutions P121K, N126H, S212G, and M216T; or
i. the amino acids 121K, 126H, 204N, 216T, and 258A, preferably the substitutions P121K, N126H, D204N, M216T, and Q258A; or
j. the amino acids 121K, 126H, 204N, 216T, and 258N, preferably the substitutions P121K, N126H, D204N, M216T, and Q258N; or
k. the amino acids 121K, 126H, 204T, 212G, and 216T, preferably the substitutions P121K, N126H, D204N, S212G, and M216T; or
l. the amino acids 121K, 126H, 204T, 216T, and 258A, preferably the substitutions P121K, N126H, D204T, M216T, and Q258A; or
m. the amino acids 121K, 126H, 204T, 216T, and 258N, preferably the substitutions P121K, N126H, D204T, M216T, and Q258N; or
n. the amino acids 121K, 126H, 212G, 216T, and 258A, preferably the substitutions P121K, N126H, S212G, M216T, and Q258A; or
o. the amino acids 121K, 126H, 212G, 216T, and 258N, preferably the substitutions P121K, N126H, S212G, M216T, and Q258N; or
p. the amino acids 121K, 126H, 204N, 212G, 216T, and 258A, preferably the substitutions P121K, N126H, D204N, S212G, M216T, and Q258A; or
q. the amino acids 121K, 126H, 204T, 212G, 216T, and 258A, preferably the substitutions P121K, N126H, D204T, S212G, M216T, and Q258A; or
r. the amino acids 121K, 126H, 204T, 212G, 216T, and 258N, preferably the substitutions P121K, N126H, D204T, S212G, M216T, and Q258N; or
s. a set of amino acid substitutions specified in Table 1.

11. The variant polypeptide of any one of claims 1-10, wherein the variant polypeptide comprises the amino acids 121K, 126H, 216T, and 258N/A; preferably the amino acid substitutions P121K, N126H, M216T, and Q258N/A.

12. The variant polypeptide of any one of claims 1-11 further comprises the amino acids
75C/V, 114T, 137E, 141T, 142D, 146R, 157G, 204C, 211W, 253Q, 267R, and 341P; or
D35Y, G70E, S80P, T161P, N176P, L179F, K180N, S187P, K276M, T277A, E315G, and A380P.

13. The variant polypeptide of any one of claims 1-12 having an increased IP4 degradation activity, and/or having a ratio of IP4 to IP6 activity of higher than 1.3, compared to the SEQ ID NO: 2.

14. A recombinant host cell comprising genetic elements that allow producing at least one variant polypeptide of any one of claims 1-13, wherein the host cell is preferably selected from the group consisting of
a. filamentous fungal cells from Division Ascomycota, Subdivision Pezizomycotina; preferably from the group consisting of members of the Class Sordariomycetes or Eurotiomycetes, Subclass Hypocreomycetidae or Sordariomycetidae or Eurotiomycetidae, Orders Hypocreales or Sordariales or Eurotiales, Families Hypocreacea or Nectriacea or Chaetomiaceae or Aspergillaceae, Genera *Trichoderma* (anamorph of *Hypocrea)* or *Fusarium* or *Acremonium* or *Humicola* or *Thermothelomyces* or *Myceliophthora* or *Aspergillus;*
more preferably from the group consisting of species *Trichoderma reesei (Hypocrea jecorina), T. citrinoviridae, T. longibrachiatum, T. virens, T. harzianum, T. asperellum, T. atroviridae, T. parareesei, Fusarium oxysporum, F. gramineanum, F. pseudograminearum, F. venenatum, Acremonium (Cephalosporium) chrysogenum, Humicola insolens, H. grisea, Thermothelomyces thermophilus, Myceliophthora thermophila, Aspergillus niger, A. niger* var. *awamori* and *A. oryzae;*
b. bacterial cells, preferably gram-positive Bacilli such as *B*. *subtilis, B*. *licheniformis, B*. *megaterium, B*. *amyloliquefaciens, B*. *pumilus,* gram negative bacteria such as *Escherichia coli,* actinomycetales such as *Streptomyces* sp.; and
c. yeasts, such as *Saccharomyces cerevisiae, Schizosaccharomyces pombe, Pichia pastoris, Yarrowia lipolytica;* and
more preferably the host cell is selected from filamentous fungal cells such as *Trichoderma,* or from gram-positive Bacilli such as *Bacillus;*
most preferably from *Trichoderma* reesei or from *Bacillus subtilis* or *B*. *pumilus* or *B*. *licheniformis* or *B*. *amyloliquefaciens.*

15. A recombinant host cell comprising genetic elements configured to produce at least one variant polypeptide of any one of claims 1-13, and wherein the host cell is a transgenic plant cell.

16. An enzyme composition comprising the variant polypeptide of any one of claims 1-13.

17. A use of the variant polypeptide of any one of claims 1-13 or the enzyme composition of claim 16 in the manufacturing of feedstuff or foodstuff, feed additive, a dietary supplement, or a pharmaceutical.

18. A method of manufacturing the variant polypeptide of any one of claims 1-13 comprising:
a. providing a polynucleotide comprising genetic elements arranged to produce the variant of claims 1-13; and
b. expressing the polynucleotide in a recombinant host cell, preferably in a recombinant host cell of claim 14 or 15.

19. An animal feed comprising the variant polypeptide of any one of claims 1-13 or the enzyme composition of claim 16, and at least one protein source of plant origin, and
a. optionally at least one further enzyme selected from protease, amylase, phytase, xylanase, endoglucanase, beta-glucanase, mannanase, cellulase, or a combination thereof; and
b. optionally at least one carrier or ingredient selected from maltodextrin, flour, salt, sodium chloride, sulphate, sodium sulphate, or a combination thereof.

20. A feed supplement comprising the variant polypeptide of any one of claims 1-13 or the enzyme composition of claim 16; and
a. optionally at least one further enzyme selected from protease, amylase, phytase, xylanase, endoglucanase, beta-glucanase, mannanase, cellulase, or a combination thereof; and
b. optionally at least one carrier or ingredient selected from maltodextrin, flour, salt, sodium chloride, sulphate, sodium sulphate, minerals, amino acids, prebiotics, probiotics, vitamins, or a combination thereof.

21. A method of degrading or modifying material containing phytic acid or phytate, comprising treating said material with an effective amount of the variant polypeptide of any one of claims 1-13 or the enzyme composition of claim 16.
